# EUROPÄISCHE PATENTANMELDUNG

(11) **EP 1 256 312 A1**
(43) Veröffentlichungstag der Anmeldung: **13.11.2002**
(21) Anmeldenummer: 01110969.1
(22) Anmeldetag: 07.05.2001
(51) Int. Cl.: A61B 5/00, A61N 1/372

(54) **Anordnung zur Überwachung und Lokalisierung von Patienten**

(71) Anmelder: Trion AG, 8604 Volketswil (CH)
(72) Erfinder: Carlson, Sven-Erik, 8704 Herrliberg (CH); Zünd, Gregor, 8704 Herrliberg (CH)
(74) Vertreter: Troesch Scheidegger Werner AG

(57) **Zusammenfassung**

Für das Überwachen einer Person mit Herz-Kreislaufstörungen wird eine Anordnung vorgeschlagen, welche
- mindestens einen Messensor (3) zum Erfassen des Kreislaufzustandes der Person (1) aufweist sowie
- eine Logiksteuerung für das Feststellen von Unregelmässigkeiten der durch den Messensor erfassten Daten,
- eine Sende- und Empfangseinrichtung (5) für Sprache und/oder Daten, um mindestens einen Dritten (9) anzuwählen und an diesen Daten zu übertragen, sowie
- ein Ortungssystem-Modul, mittels welchem der Standort der Person an den Dritten übermittelt wird.

## Beschreibung

Die vorliegende Erfindung betrifft eine Anordnung zum Überwachen einer Person, insbesondere mit Herz-Kreislaufstörungen gemäss dem Oberbegriff nach Anspruch 1, ein Verfahren zum Ueberwachen eines Patienten, insbesondere mit Herz-Kreislaufstörungen sowie die Verwendung einer Anordnung.

Bei Personen mit Herz-Kreislaufstörungen ist es eminent wichtig, dass im Falle auftretender akuter Herzprobleme sofort notwendige Massnahmen vorgenommen werden können, ansonsten innerhalb relativ kurzer Zeit irreparable Schädigungen oder gar der Tod der Person eintreten können.

Bei Ueberwachung eines Patienten in einer Intensivstation, beispielsweise nach einem Herzinfarkt, bei ernsthafter Herzerkrankung oder nach einer Herzoperation ist jederzeit gewährleistet, dass bei auftretenden Problemen sofort die notwendige Hilfeleistung erfolgen kann.

Bereits beim Verlegen eines Patienten in die "normale" Bettenabteilung eines Spitals ist eine permanente Ueberwachung erschwert bzw. nur bedingt möglich. Wohl kann der Patient selbst im Falle von Problemen einen Alarm auslösen oder aber an Patienten angeschlossene Ueberwachungsgeräte können im Falle von Unregelmässigkeiten ein entsprechendes Signal erzeugen. Neuerdings bekannt ist ein Patientenüberwachungssystem in Spitälern, bei welchem bei Auftreten von Problemen automatisch Alarmsignal an beispielsweise eine Aufsichtsperson, wie eine Stationsschwester, weitergeleitet wird. Allerdings funktioniert diese Überwachung nur, falls sich der Patient in seinem Zimmer aufhält, da dieses System die Ortung des Aufenthaltes des Patienten nicht garantiert.

Beim Entlassen des Patienten aus dem Spital oder aber auch bei nicht hospitalisierten Patienten wird die Ueberwachungsproblematik verschärft. Es besteht praktisch nur noch die Möglichkeit, dass der Patient selbst bei auftretenden Problemen einen Alarm auslösen kann, beispielsweise durch Betätigen eines auf dem Patienten getragenen Drucksensors, mit welchem beispielsweise ein Telefonalarm ausgelöst werden kann. Vielfach ist der Patient aber dazu nicht mehr in der Lage und zudem weiss eine den Alarm entgegennehmende Person nicht, wo genau sich der Patient aufhält. Dies vor allem dann, wenn der Patient selbst sich nicht mehr mitteilen kann.

Es ist daher eine Aufgabe der vorliegenden Erfindung, eine Anordnung zu schaffen mittels welcher eine Person, insbesondere mit Herz-Kreislaufproblemen, jederzeit überwacht und geortet werden kann.

Vorgeschlagen wird eine Anordnung gemäss dem Wortlaut, insbesondere nach Anspruch 1.

Vorgeschlagen wird eine Anordnung zum Ueberwachen, welche mindestens die folgenden Komponenten aufweist:
- mindestens einen Messsensor an der Person zum Erfassen des Kreislaufzustandes,
- eine Logiksteuerung für den Sensor um festzustellen, ob sich die Messwerte innerhalb oder ausserhalb eines definierten Normalbereiches befinden,
- eine Sende- und Empfangseinrichtung für Sprache und/ oder Daten, um mindestens einen Dritten anzuwählen und an diesen Daten zu übertragen, sowie
- ein Positionierungssystem, das die genaue Ortung ermöglicht, wie beispielsweise ein GPS (Global Positioning System)-Modul, mittels welchem der Standort an den Dritten übermittelt wird.

Der oder die Messsensoren, welche den Kreislaufzustand der Person überwachen, erfassen vorteilhafterweise die Herzfrequenz, den Blutdruck, die Sauerstoffsättigung und gegebenenfalls weitere Faktoren, wie Körpertemperatur, Atemfrequenz, etc. Der oder die Sensoren sind so am, auf oder im Körper anzuordnen, dass sie eine maximale Bewegungsfreiheit und eine minimale Beeinträchtigung des normalen Lebens gewährleisten. Vorteilhaft werden alle Sensoren in einer einzigen Sensoreinheit angeordnet, welche beispielsweise als Armband oder als Chip subkutan getragen werden kann. Selbstverständlich kann diese Sensoreinheit auch an irgendeiner anderen Körperstelle angeordnet werden.

Der oder die Sensoren werden von einer Logik gesteuert, die kontrolliert, ob sich die Messwerte innerhalb oder ausserhalb des durch einen Arzt der Person bzw. des Patienten definierten Normalbereiches befinden. Werden Messwerte ausserhalb des Normalbereiches festgestellt, gibt die Sensoreinheit mittels einer Drahtverbindung oder vorzugsweise einer drahtlosen Verbindung, wie beispielsweise einem sogenannten Radiotransceiver, an eine Daten-, Sende- und Empfangseinrichtung für Sprache und/oder Daten, welche die Person auf sich trägt, den Befehl, automatisch eine Verbindung zu mindestens einem Empfänger, wie beispielsweise einer vorprogrammierten Telefonnummer oder Internetadresse, herzustellen.

Bei dieser Sende- und Empfangseinrichtung kann es sich um ein mobiles Telekommunikationsgerät handeln, wie beispielsweise um ein sogenanntes GSM-Telefon (Global System for Mobile communication), ein UMTS-Gerät (Universal Mobile Telecommunication System) etc., welche Geräte allgemein üblich als drahtlose Kommunikationsmittel bzw. als Ersatz für stationäre Telefonate verwendet werden. Grundsätzlich können irgendwelche mobilen Telekommunikationsgeräte verwendet werden, welche drahtlos Daten und/oder Sprachinformationen übertragen, sei dies via ein Telekommunikationsnetz oder via Internet. Gegebenenfalls muss bei diesem mobilen Telefon eine Zusatzeinheit vorgesehen werden, enthaltend eine Einrichtung für die drahtlose Kommunikation mit der Sensoreinheit sowie eine Steuerungselektronik für das automatische Anwählen eines Empfängers. Für die drahtlose Kommunikation zwischen Sensoreinheit und Uebertragungseinrichtung, wie dem erwähnten GSM-Telefon, drängt sich eine Datenkommunikation im Radiofrequenzbereich auf, wie beispielsweise die neuerdings für lokale Sprach- und Datenkommunikation verwendete sogenannte "Bluetooth"-Technologie, welche auf einfachste Art und Weise und unter Verwendung kleinster Module einen drahtlosen Informationsaustausch zwischen mehreren Geräten ermöglicht. Diese Bluetooth-Technologie wird neuerdings auch bei den erwähnten GSM-Telefongeräten verwendet, womit das Anordnen der erwähnten Zusatzeinheit überflüssig wird.

Damit nun, wie oben erwähnt, ein Empfänger, wie beispielsweise eine medizinische Fachperson oder ein diensttuender Arzt in einem Spital nebst der Tatsache, dass bei der zu überwachenden Person ernsthafte, gesundheitliche Probleme auftreten auch weiss, wo sich die Person aufhält, wird nun erfindungsgemäss vorgeschlagen, ein Ortungssystem wie die sogenannte GPS-Technologie zu verwenden. Neuerdings werden auf dem Markt Mobiltelefone angeboten, welche zusätzlich sogenannte GPS(Global Positioning System)-Navigation ermöglichen. Damit werden nun zusätzlich zu den den Herz-Kreislaufzustand charakterisierenden Daten ebenfalls die Positionskoordinaten der Person an den Empfänger übermittelt, womit dieser sofort weiss, wo sich die Person aufhält. Der Empfänger kann entweder selbst sofort den Patienten aufsuchen oder aber beispielsweise eine Notfalldienststelle oder einen Notfallarzt aufbieten, welcher sich in der Nähe der Person aufhält.

Ein weiterer Vorteil bei der Verwendung eines Mobiltelefons für die Uebertragung der Daten vom Patienten zum Empfänger liegt darin, dass eine Kommunikation in beide Richtungen möglich wird. Speziell durch die neuentwickelten Technologien wie UMTS, GPRS (General Packet Radio Service) usw. ist es möglich, von einem Mobiltelefon gleichzeitig Sprach- und Datenkommunikation mit einer externen Stelle wie einem weiteren Telefonanschluss oder einem Modem via Internet etc. zu führen. Der Empfänger, wie beispielsweise der Hausarzt oder eine medizinische Fachperson, kann versuchen mit dem Patienten zu kommunizieren, falls dieser bei Bewusstsein ist und sprechen kann. Mit anderen Worten ermöglicht die Verwendung eines Mobiltelefons dem Empfänger, in direkten Kontakt mit der überwachten Person zu treten. Grundsätzlich aber erfolgt die Datenkommunikation direkt und automatisch bei Über- oder Unterschreiten einer vorgegebenen Alarmgrenze und durch entsprechende Verbindungsaufnahme.

Bei Kommunikation bzw. Datenaustausch in beiden Richtungen ist es zusätzlich möglich, dass der Empfänger Daten bei der Sensoreinheit abfragen kann, um beispielsweise die Herzfrequenz über eine gewisse Zeitdauer verfolgen zu können. Diese Daten können beispielsweise auf einem Bildschirm veranschaulicht werden, so dass der Zustand des Patienten optimal dargestellt werden kann.

Durch das zusätzliche Wissen des präzisen Standortes des Patienten kann anhand der dem Empfänger zur Verfügung stehenden Daten die optimale, dem Zustand des Patienten entsprechende Hilfsaktion ausgelöst werden.

Bekanntlich gibt es weltweit immer mehr Patienten mit Herz-Kreislaufstörungen. Diese Patienten befürchten ausserhalb eines Spitals, das ihre Situation kurzfristig und ohne Vorwarnung lebensbedrohend werden könnte. Durch die erfindungsgemäss vorgeschlagene Anordnung wird nun solchen Patienten die Möglichkeit angeboten, automatisiert eine Drittperson, wie beispielsweise eine medizinische Fachperson, zu benachrichtigen, falls sich der Gesundheitszustand lebensgesundheitsbedrohlich verändert. Das erfindungsgemäss vorgeschlagene System verbessert aufgrund des erhöhten Sicherheitsgefühls die Lebensqualität des Patienten. Das System verringert die Reaktionszeit zwischen dem Eintreten der gesundheitlichen Veränderung und der medizinischen Behandlung durch:
- das wesentlich schnellere Eintreffen von Rettungsdiensten beim Patienten,
- die Möglichkeit der Beurteilung des Gesundheitszustandes aufgrund der via Telekommunikation übermittelten Daten.

Das System gewährleistet zudem optimalen Einsatz von Material und Personal beim Ausrücken, da eine Erstdiagnose bekannt ist und eine Lokalisation bereits stattgefunden hat.

Das System bzw. die erfindungsgemäss vorgeschlagene Anordnung kann dazu beitragen, Funktionsschäden bei Ueberlebenden zu vermindern und unter Umständen sogar lebensrettend zu wirken.

Die erfindungsgemäss vorgeschlagene Anordnung ist sinnvoll - notwendig, insbesondere beim Ueberwachen bei Personen mit Herzproblemen und/oder Kreislaufstörungen. Selbstverständlich ist die erfindungsgemässe Anordnung geeignet für die Ueberwachung von Patienten mit anderen Leiden, doch drängt sich eine ständige Ueberwachung mit der Möglichkeit eines sofortigen Eingreifens speziell bei Patienten mit Herz-Kreislaufstörungen auf.

Die Erfindung wird nun anschliessend beispielsweise und unter Bezug auf die beigefügten Figuren näher erläutert.

Dabei zeigen:
- Fig. 1: anhand einer schematischen Darstellung das Prinzip und die Funktionsweise der vorliegenden Erfindung, und
- Fig. 2: anhand eines weiteren Schemas die einzelnen Elemente und das Funktionsprinzip der vorliegenden Erfindung.

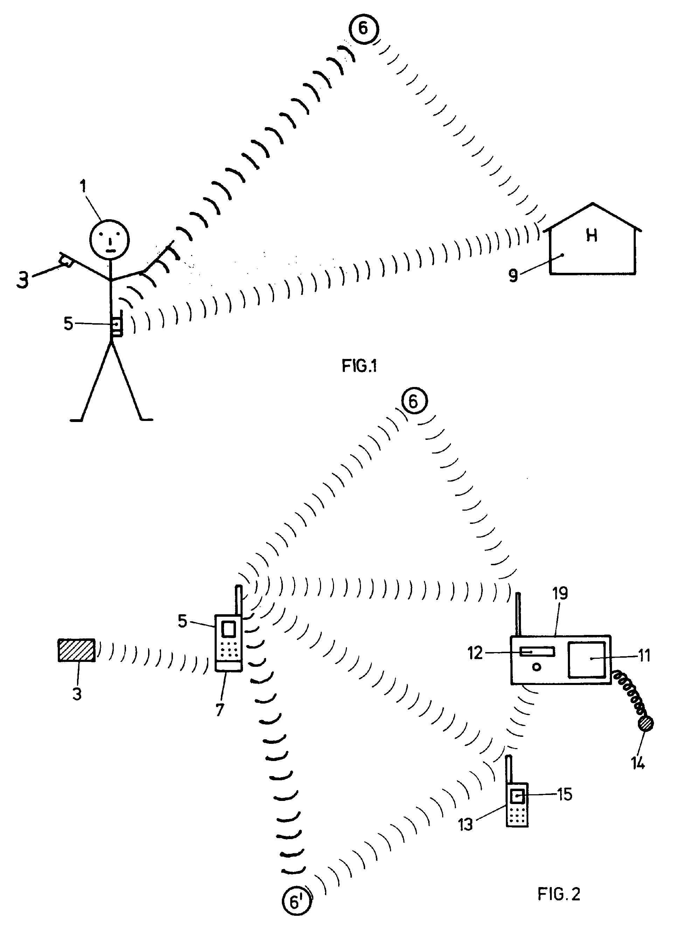

Figur 1 zeigt anhand einer schematischen Darstellung die Funktionsweise der vorliegenden Erfindung.

Bei einer Person 1 handelt es sich um eine Person mit Herz-Kreislaufstörungen. Dabei kann es sich um einen Patienten handeln, welcher sich in ärztlicher Behandlung befindet, oder aber um eine Person, welche vor kurzem aus einem Spital entlassen worden ist, in welchem er sich aufgrund beispielsweise eines Herzinfarktes aufgehalten hat, oder in welchem Spital er sich einer Herzoperation unterzogen hat. Wesentlich ist, dass bei der Person 1 der Verdacht besteht, dass kurzfristig Herzprobleme auftreten können, welche für die Person 1 eine ernsthafte Bedrohung darstellen. Aus diesem Grunde ist es wichtig, dass die Person 1 ständig unter ärztlicher Kontrolle steht, d.h. dass ständig der Gesundheitszustand der Person 1 überwacht werden kann.

Dies geschieht nun mittels einer Sensoreinheit 3, welche einen oder mehrere Sensoren aufweisen kann, mittels welcher beispielsweise die Herzfrequenz, der Blutdruck, die Blutsättigung, die Körpertemperatur und gegebenenfalls weitere gesundheitsrelevanten Faktoren überwacht werden können. In der Sensoreinheit 3 weiter angeordnet ist eine Logiksteuerung, welche ständig kontrolliert, ob sich die Messwerte innerhalb oder ausserhalb eines durch einen Arzt des Patienten definierten Normalbereiches befinden. Werden Messwerte ausserhalb des Normalbereiches festgestellt, gibt die Sensoreinheit mittels einer Drahtverbindung oder mittels einer drahtlosen Verbindung, wie vorzugsweise einem sogenannten Radio-Transceiver, ein Befehlssignal an ein Mobiltelefon 5 ab, welches sich ebenfalls auf dem Patienten befindet. Aufgrund dieses Signales wird beim Mobiltelefon, bei welchem es sich beispielsweise um ein sogenanntes GSM-Telefon (global system for mobile communication) handelt, ein Wählimpuls ausgelöst, mittels welchem ein oder mehrere Empfänger angewählt werden. Beim Empfänger kann es sich beispielsweise um eine Rettungszentrale 9 handeln, welche beispielsweise von einer medizinischen Fachperson bedient wird. Bei Verbindungsaufbau durch die medizinische Fachperson werden dieser über die Verbindung vom Mobiltelefon 5 zum Anschluss im Spital 9, wie eine Telefonstation oder ein Internetanschluss, die von der Messeinheit gemessenen Daten übermittelt, so dass die Fachperson aufgrund dieser Daten und der Identifikation des Patienten, welche ebenfalls durch das Mobiltelefon 5 ermöglicht wird, sofort eine Beurteilung des Gesundheitszustandes erstellen kann und welche Massnahme einzuleiten sind.

Ganz wichtig ist natürlich, dass der medizinischen Fachperson die Positions-Koordinaten des Patienten 1 bekannt sind, damit sie weiss, wo sich dieser aufhält. Dies lässt sich beispielsweise mittels des sogenannten und bereits weit verbreiteten GPS-Systems (Global Positioning System) feststellen, indem vom Mobiltelefon 5 zusätzlich zur Datenübertragung auch die Positionskoordinaten via Satelliten 6 mittels des erwähnten GPS-Systems übertragen werden.

Nun kann im Rettungszentrum entschieden werden, ob eine Equipe des Spitals oder eine auswärtige Stelle aufzubieten ist, um beim Patienten die notwendige Hilfe zu leisten.

Anhand des Schemas von Figur 2 soll die vorliegende Erfindung bzw. das Funktionsprinzip noch näher erläutert werden.

Wie bereits erwähnt, werden von der Sensoreinheit 3 gemessene Daten im Falle des Abweichens aus einem vorgegebenen Messbereich beispielsweise drahtlos an eine Mobiltelefoneinheit 5 übertragen. Für die Kommunikation zwischen Sensoreinheit 3 und dem Mobiltelefon 5 kann eine Drahtverbindung bestehen wie auch eine drahtlose, wie beispielsweise mittels Infrarot, indem sowohl an der Sensoreinheit wie dem Mobiltelefon eine Infrarot-Schnittstelle für Datentransfer vorgesehen ist, und weiter geeignet ist insbesondere Datentransfer im Radiowellenbereich, wie beispielsweise mittels der sogenannten "Bluetooth"-Technologie. Diese Technologie gewährleistet den Informationsaustausch zwischen Geräten ohne Verwendung irgendwelcher Kabelverbindungen. Neuerdings wird diese "Bluetooth"-Technologie, beispielsweise im Zusammenhang mit sogenannten Note-Books oder Laptop-Personalcomputers verwendet, indem diese mobilen Personalcomputer jederzeit drahtlos innerhalb eines gewissen Bereiches mit einer Zentraleinheit verbunden sind, und somit jeder Zeit eine drahtlose Datenkommunikation möglich ist. Aber auch im Bereich von Mobiltelefonen wird die Verwendung der erwähnten "Bluetooth"-Technologie vorgeschlagen. Je nach dem wie die Datenkommunikation zwischen Sensoreinheit 3 und Mobiltelefon 5 erfolgt, muss an letzterem eine Zusatzeinheit 7 angeordnet werden, welche eine Einrichtung für die drahtlose Kommunikation mit der Sensoreinheit beinhaltet sowie eine Steuerungselektronik.

Im Falle der erwähnten "Bluetooth"-Technologie, welche neuerdings auch in Mobiltelefonen integriert ist, entfällt die Notwendigkeit des Anordnens der erwähnten Zusatzeinheit 7.

Vom Mobiltelefon 5 wird im Falle des Abweichens der erwähnten Messdaten aus einem vorgegebenen, definierten Bereich automatisch ein Empfänger angewählt, wie beispielsweise ein Telekommunikationsgerät 19, welches mit einer Datenerfassungs- und Auswertungseinheit verbunden ist. An dieser werden an einer Anzeige 12, die von der Sensoreinheit 3 gemessenen Daten wieder gegeben, so dass eine bei der Empfängereinheit 19 diensttuende Person sofort eine Zustandsbeurteilung über die Gesundheit des Patienten vornehmen kann. Mittels der über einen Satelliten 6 mittels des GPS-Systems übertragenen Positionskoordinaten, wo sich der Patient bzw. das Mobiltelefon 5 befindet, kann zudem die diensttuende Person beispielsweise an einem Bildschirm 11 sofort den Standort des Patienten feststellen. Somit kann praktisch verzögerungsfrei beim Auftreten von Gesundheitsproblemen des Patienten die diensttuende medizinische Fachperson sofort die notwendigen Massnahmen veranlassen, um dem Patienten zu helfen. Zusätzlich ist es möglich, beispielsweise mittels Telefon 12 mit dem Patienten in sprachlichen Kontakt zu treten, da ja durch das Verwenden der Mobiltelefoneinheit 5 eine Kommunikation möglich ist. Ist der Patient ansprechbar, so kann sich beispielsweise die medizinische Fachperson bei diesem über sein Befinden resp. über seine Eindrücke der Situation erkundigen.

Von der Rettungszentrale kann auch ein weiteres Mobiltelefon 13 verständigt werden, welches beispielsweise von dem den Patienten behandelnden Arzt getragen wird. An einem Display 15 des Mobiltelefons 13 können ebenfalls die von der Sensoreinheit 3 gemessenen Messdaten abgelesen werden, welche von der Rettungszentrale weiter an das Mobiltelefon geleitet werden können. Der das Mobiltelefon 13 tragende behandelnde Arzt kann nun seinerseits mit dem Patienten in sprachlichen Kontakt treten. Selbstverständlich ist es auch möglich, dass der Datentransfer vom Patienten direkt an das Mobiltelefon 13 des behandelnden Arztes übertragen wird, und auch der behandelnde Arzt kann ggf. den Standort des Patienten ermitteln, indem diesem über Satelliten 6' mittels GPS die Koordinaten übermittelt werden. In der Regel aber ist der Kontakt bzw. die Datenübertragung an eine Rettungszentrale obligatorisch, und die Mitteilung an den behandelnden Arzt erfolgt je nach Umständen.

Mit dem erfindungsgemäss vorgeschlagenen Ueberwachungssystem bzw. der Anordnung ist es aber auch möglich, dass beispielsweise der Hausarzt von Zeit zu Zeit über die Datenkommunikationskette Daten bei der Sensoreinheit 3 abruft, um sich so ein Bild über den Gesundheitszustand des Patienten zu machen. So ist es beispielsweise wichtig zu wissen, ob nach Auslösen eines Alarmes beispielsweise die Herzfrequenz wieder normal ist, oder ob gar ein Herzstillstand vorliegt. Daraus ergibt sich die Dringlichkeit der zu ergreifenden Massnahmen.

Selbstverständlich handelt es sich bei den beiden in den Figuren 1 und 2 dargestellten Schemas lediglich um Beispiele, um die vorliegende Erfindung näher zu erläutert. Die in den Schemas gewählten Elemente sowie die beschriebenen Uebertragungstechnologien richten sich nach den heute üblich verwendeten Technologien und Möglichkeiten. Insbesondere sind Mobiltelefone mit integriertem GPS-System erst seit kurzem auf dem Markt und werden erst von wenigen Herstellern angeboten, wie beispielsweise der finnischen Firma Benefon OY. Es ist aber davon auszugehen, dass derartige Geräte in Bälde auch von anderen Herstellern angeboten werden. Auch bezüglich der "Bluetooth"-Technologie ist ergänzend zu erwähnen, dass diese Technologie erst bei wenigen Geräten und Systemen verwendet wird. Aber auch diese Technologie bzw. artverwandte Technologien werden insbesondere dem Bereich der Datenverarbeitung und Datenkommunikation in Zukunft massgeblich beeinflussen, so dass diese Technologien selbstverständlich im Zusammenhang mit der vorliegenden Erfindung entsprechend Verwendung finden können. Auch bezüglich Messensor sowie Logiksteuerung sind verschiedene Ausführungen denkbar. So können beispielsweise die Messensoren in einem Fingerring angeordnet werden und die Auswertelektronik bzw. Logiksteuerung in einer Armbanduhr, wobei die Datenübertragung via Infrarot-Schnittstelle erfolgen kann. Oder aber Messensor und Auswertelektronik sowie Logiksteuerung können allesamt in einer Armbanduhr oder generell in einem Armband angeordnet sein. Schliesslich können sowohl Sensor wie Auswertelektronik und Logiksteuerung an irgendeiner anderen geeigneten Körperstelle und unter Verwendung eines geeigneten Trägers angeordnet werden.

Es ist aber auch möglich, den Messensor am Ohr anzuordnen, beispielsweise in einem Ohrclip oder integriert in einem Hörgerät. Je nach dem kann es sowohl beim Anordnen der Messensoren in einem Fingerring oder aber am Ohr vorteilhaft sein, wenn permanent Daten vom Messensor an die Auswert- und Überwachungslogik gesendet werden, speziell dann, wenn diese örtlich beabstandet vom Messensor angeordnet ist, wie beispielsweise in einem Armband, oder aber direkt im oder am Telekommunikationsgerät, wie beispielsweise dem Mobiltelefon.

In diesem Sinne beschränkt sich die vorliegende Erfindung nicht auf die im Zusammenhang mit den beiden Figuren 1 und 2 angeführten Technologien und Ausführungsbeispiele der einzelnen Module, sondern umfasst auch solche, die momentan erst in Entwicklung und auf dem Markt noch nicht erhältlich sind.

## Patentansprüche

1. Anordnung zum Ueberwachen einer Person mit Herz-Kreislaufstörungen, **gekennzeichnet durch**
- mindestens einen Messsensor (3) zum Erfassen des Kreislaufzustandes der Person (1),
- eine Logiksteuerung für das Feststellen von Unregelmässigkeiten der **durch** den Messsensor erfassten Daten,
- eine Sende- und Empfangseinrichtung (5) für Sprache und/oder Daten, um mindestens einen Dritten (9, 13, 19) anzuwählen, und an diesen Daten zu übertragen, sowie
- ein Ortungssystem-Modul, mittels welchem der Standort der Person an den Dritten übermittelt wird.

2. Anordnung, insbesondere nach Anspruch 1, **dadurch gekennzeichnet, dass** ein oder mehrere Messsensoren vorgesehen sind zum Erfassen der Herzfrequenz, des Blutdruckes, der Sauerstoffsättigung und/oder der Körpertemperatur.

3. Anordnung, insbesondere nach einem der Ansprüche 1 oder 2, **dadurch gekennzeichnet, dass** als Sende- und Empfangseinheit (5) ein Telekommunikationsgerät wie ein Mobiltelefon dient, welches als Zusatzmodul ein automatisch auslösbares Anwählorgan aufweist, welches auf ein Signal der Logiksteuerung hin auslösbar ist.

4. Anordnung, insbesondere nach einem der Ansprüche 1 bis 3, **dadurch gekennzeichnet, dass** an der Sende- und Empfangseinheit (5) eine Sensor- und Steuerungselektronik angeordnet ist, welche mit dem Anwählorgan derart verbunden ist, dass eine oder mehrere vorprogrammierte Telefonnummern und/oder Internetadressen angewählt werden, und dass von der Sendeeinheit nebst Messdaten auch Positionskoordinaten wie GPS (Global Positioning System)-Koordinaten an den Dritten übermittelt werden.

5. Anordnung, insbesondere nach einem der Ansprüche 1 bis 4, **dadurch gekennzeichnet, dass** für die Uebermittlung der Daten vom Messsensor (3) bzw. der Logiksteuerung zur Uebertragungseinheit (5) Datenkommunikation im Radiowellen-Bereich wie sogenannte "Bluetooth"-Technologie-Komponenten verwendet werden.

6. Anordnung nach einem der Ansprüche 1 bis 5, **dadurch gekennzeichnet, dass** beim Dritten eine Einrichtung (11, 12, 15) vorgesehen ist, an welcher die vom Messsensor gemessenen Daten dargestellt bzw. visualisiert werden können sowie der Standort der zu überwachenden Person.

7. Anordnung nach einem der Ansprüche 1 bis 6, **dadurch gekennzeichnet, dass** die Sende- und Empfangseinrichtung sowie die beim Dritten angeordnete Einrichtung dergestalt sind, dass Daten- und Sprachkommunikation in beiden Richtungen möglich ist, um ein Kommunizieren zwischen der Person und dem Empfänger zu ermöglichen.

8. Verfahren zum Ueberwachen einer Person mit Herz-Kreislaufstörungen, **dadurch gekennzeichnet, dass**
- mittels mindestens eines Messsensors (3) an der Person (1) der Kreislaufzustand überwacht wird,
- mittels einer Logiksteuerung Unregelmässigkeiten der erfassten Daten festgestellt werden,
- mindestens im Falle von Unregelmässigkeiten mittels einer Sende- und Empfangseinrichtung für Sprache und/oder Daten (5) mindestens ein Dritter angewählt wird und Daten übertragen werden, sowie
- mittels eines Ortungs- oder Navigationssystems, wie GPS (Global Positioning System), dem Dritten die Position der Person übermittelt wird.

9. Verfahren, insbesondere nach Anspruch 8, **dadurch gekennzeichnet, dass** die Uebertragung der Daten vom Messsensor zur Sende- und Empfangseinrichtung mittels Radiowellen wie beispielsweise sogenannter "Bluetooth"-Technologie erfolgt.

10. Verfahren, insbesondere nach einem der Ansprüche 8 oder 9, **dadurch gekennzeichnet, dass** als Sende- und Empfangseinrichtung (5) ein GSM-Gerät (global system for mobile communication), ein GPRS-Gerät (general packet radio service), ein UMTS-Gerät (universal mobile telecommunication system) etc. verwendet wird, welches aufgrund eines Signals durch die Logiksteuerung automatisch mindestens einen Dritten anwählt.

11. Verfahren, insbesondere nach einem der Ansprüche 8 bis 10, **dadurch gekennzeichnet, dass** die Daten- und Sprachkommunikation zwischen Sende- und Empfangseinrichtung (5) und dem Dritten in beiden Richtungen möglich ist, damit der Dritte mit der Pperson Kontakt aufnehmen kann, bzw. gegebenenfalls Daten beim Messsensor an der Person ablesen bzw. den Messsensor beeinflussen kann oder andere, beim Patienten befindliche, Einrichtungen.

12. Verwendung der Anordnung nach einem der Ansprüche 1 bis 7 für die Ueberwachung einer Person mit Herz-Kreislaufstörungen.
